Europäisches Patentamt

(19) European Patent Office

Office européen des brevets

(11) Veröffentlichungsnummer : **0 004 104**
**B2**

(12) **NEUE EUROPÄISCHE PATENTSCHRIFT**

(45) Veröffentlichungstag der neuen Patentschrift :
15.04.87

(21) Anmeldenummer : 79200085.3

(22) Anmeldetag : 19.02.79

(51) Int. Cl.⁴ : **C 07 D211/74**

(54) Verfahren zur Herstellung von 2,2,6,6-Tetramethylpiperidon-(4).

(30) Priorität : **20.02.78 DE 2807172**

(43) Veröffentlichungstag der Anmeldung :
**19.09.79 Patentblatt 79/19**

(45) Bekanntmachung des Hinweises auf die Patenterteilung : **24.03.82 Patentblatt 82/12**

(45) Bekanntmachung des Hinweises auf die Entscheidung
über den Einspruch : **15.04.87 Patentblatt 87/16**

(84) Benannte Vertragsstaaten :
**BE CH DE FR GB IT LU NL SE**

(56) Entgegenhaltungen :
**DE-A- 2 429 745**
**DE-A- 2 429 746**
**DE-A- 2 429 936**
**DE-A- 2 429 937**
**US-A- 3 904 625**
**SU-Zeitschrift Khimicheskaya promyshlenost**
**(Chemische Industrie) Jahrg. 1967 Heft 7, S. 500-503**
**Die Akte enthält technische Angaben, die nach dem**
**Eingang der Anmeldung eingereicht wurden und die**
**nicht in dieser Patentschrift enthalten sind.**

(73) Patentinhaber : **HÜLS AKTIENGESELLSCHAFT**
**Patentabteilung / PB 15 - Postfach 13 20**
**D-4370 Marl 1 (DE)**

(72) Erfinder : **Disteldorf, Josef, Dr.**
**Am Sengenhoff 2a**
**D-4690 Herne 1 (DE)**
Erfinder : **Hübel, Werner, Dr.**
**Birnenbruchstrasse 34**
**D-4690 Herne 1 (DE)**
Erfinder : **Broschinski, Lothar, Dr.**
**Lünenbrink 18**
**D-4760 Werl (DE)**
Erfinder : **Kriebel, Günter, Dr.**
**Gaussstrasse 15**
**D-4690 Herne 2 (DE)**

(74) Vertreter : **Steil, Hanna, Dipl.-Chem. et al**
**RSP PATENTE - PB 15 Postfach 1320**
**D-4370 Marl 1 (DE)**

EP 0 004 104 B2

## Beschreibung

Es sind verschiedene Verfahren zur Herstellung von 2,2,6,6-Tetramethylpiperidon-(4) (Triacetonamin oder auch 2,2,6,6-Tetramethyl-4-oxo-piperidin genannt) bekannt. Sie bestehen meist — wie in DE-AS 2 352 127 angeführt —, darin, dass zunächst hergestellt Vorkondensate des Acetons, wie Phoron und Acetonin, in einem weiteren Verfahrensschritt in das 2,2,6,6-Tetramethylpiperidon-(4) übergeführt werden. Diese Methoden besitzen jedoch teilweise den Nachteil, dass bei langen Reaktionszeiten nur geringe Ausbeuten anfallen und die in den wässrigen Laugen anfallenden Nebenprodukte ökologische Probleme darstellen.

Eine gewisse Verbesserung wurde durch Umsetzung von vorgebildetem Phoron mit wässrigem Ammoniak bei erhöhter Temperatur und erhöhtem Druck erzielt.

Weiterhin kann Triacetonamin durch Reaktion von 2,2,4,4,6-Pentamethyl-2,3,4,5-tetrahydropyrimidin (Acetonin) mit Aceton oder seinen Kondensationsprodukten, wie Diacetonalkohol, Mesityloxid oder Phoron, und Wasser entweder in der Wärme oder in Gegenwart von Protonensäuren bzw. ihrer Salze mit Ammoniak oder Aminen, oder mit basischen Ionenaustauschern oder mit Lewis-Säuren, wie Zinkchlorid oder Calciumchlorid in Gegenwart von Wasser hergestellt werden. Hierzu wird auf die DE-Offenlegungsschriften 2 429 935, 2 429 745, 2 429 936 verwiesen.

Auch kann Triacetonamin aus Acetonin in Anwesenheit von mindestens 12,5 Mol-% eines sauren Katalysators unter wasserfreien Bedingungen hergestellt werden. Alle diese Methoden besitzen also den Nachteil, dass zunächst in einem getrennten Verfahren Vorprodukte hergestellt werden müssen.

In beiden Fällen wird das als Ausgangsmaterial eingesetzte Pyrimidinderivat ebenfalls zunächst aus Aceton und Ammoniak hergestellt und nach Isolierung und Reinigung zur Herstellung von 2,2,6,6-Tetramethyl-4-oxo-piperidin eingesetzt.

Es sind auch Verfahren bekannt, die direkt von Aceton ausgehen. Bei der Verwendung von Aceton als Ausgangsprodukt ist ein zwei- und einstufiges Verfahren bekannt. Bei dem einstufigen Verfahren wird Aceton und Ammoniak in Gegenwart von wasserfreiem Calciumchlorid kondensiert (s. Asinger, Monatshefte f. Chem. 99, 1436 (1968)). Bei diesem Verfahren wird 2,2,6,6-Tetramethylpiperidon-(4) in etwa 40 prozentiger Ausbeute erhalten. Daneben fallen bei der Kondensation grössere Mengen an wässriger Calciumchloridlösung an, deren Beseitigung wegen der Verunreinigung mit organischen Substanzen und wegen der Möglichkeit der Umweltverschmutzung erhebliche Schwierigkeiten bereitet.

Gemäss SU-PS 520 357 erfolgt die Herstellung aus Aceton und Ammoniak bei erhöhter Temperatur mittels AlCl₃-Katalysatoren.

Katalysatoren wie Calciumchlorid oder Aluminiumchlorid ergeben den Nachteil, dass sie vor ihrer Wiederverwendung vom Reaktionswasser getrennt werden müssen. Sie sind deshalb nur in begrenztem Umfang für eine kontinuierliche Herstellung geeignet.

Es wurde nun überraschenderweise ein Verfahren zur Herstellung von 2,2,6,6-Tetramethylpiperidon-(4) durch Umsetzung von Aceton und Ammoniak in Gegenwart von festen sauren Katalysatoren und bei erhöhter Temperatur gefunden, das diese beschriebenen Nachteile nicht besitzt.

Gegenstand der Erfindung ist daher ein Verfahren zur einstufigen Herstellung von 2,2,6,6-Tetramethylpiperidon-(4) durch Umsetzung von Aceton und Ammoniak im Molverhältnis 2-25 : 1 in Gegenwart von festen sauren Katalysatoren bei erhöhter Temperatur, beim Eigendruck des Systems, der gegebenenfalls durch Inertgaszugabe erhöht ist, dadurch gekennzeichnet, dass man Aceton und Ammoniak bei Temperaturen im Bereich von 80-130 °C in Gegenwart von festen sauren Katalysatoren, die sowohl in den Einsatzprodukten als auch im Reaktionsgemisch praktisch unlöslich sind, umsetzt.

Bedingung für die Eignung für diese Umsetzung ist, dass der feste, saure Katalysator in dem Reaktionsmedium unlöslich ist. Für die beschriebene Umsetzung unter Verwendung von festen, sauren Katalysatoren sind sowohl solche auf anorganischer als auch organischer Basis mit aktiven Sulfonsäureresten oder ähnlich aktiven sauren Gruppen geeignet. Die chemische Beständigkeit derartiger Festbettkatalysatoren in aliphatischen und auch anderen Lösungsmitteln ist ausserordentlich hoch, so dass diese in dem Reaktionsgemisch praktisch unlöslich sind.

Beispiele für geeignete anorganische Katalysatoren sind beispielsweise saure Aluminiumhydrosilikate vom Bentonit- und/oder Montmorillonit-Typ und anorganische Ionenaustauscher auf Aluminiumsilikat-Basis vom Zeolith-, Mordenit- und/oder Erionittyp, deren Acidität ausreicht um die Reaktion zu katalysieren. Ebenfalls geeignet für diesen Zweck sind Trägermaterialien, wie beispielsweise Diatomeenerde, die zunächst mit Phosphorsäure behandelt worden sind und anschliessend einer Hitzebehandlung zwischen 700-1 100 °C unterworfen worden sind. Die Herstellung derartiger Katalysatoren ist in CA-PS 772 201 beschrieben worden. Weitere Beispiele für feste, saure Katalysatoren sind Ionenaustauscher auf organischer Basis, insbesondere solche mit einem Styrol-Divinylbenzol-Copolymeren als Matrix und mit —SO₃⁻ als Ankergruppen. Produkte dieses Typus sind beispielsweise im Handel unter der Bezeichnung « Amberlyst® 15 », « Amberlite® 200 » bzw. « Lewatit® SP 120 » erhältlich. Diese Typen sind insbesonders zum Einsatz in nichtwässrigen Lösungen bestimmt, zumal vor längerer Zeit festgestellt worden ist, dass saure Austauscher auch zur Katalyse in nichtwässrigen Lösungen verwendet können. Die festen, sauren Ionenaustauscher werden zweckmässigerweise in der Ammonium- oder Alkylammoniumform eingesetzt, wobei bevorzugt solche Amine für die Aminform eingesetzt werden, die als Zwischen- bzw. Endprodukte bei der Umsetzung von Aceton mit Ammoniak entstehen.

Solche Katalysatoren sind weder in den Ausgangsprodukten noch im wasserhaltigen Reaktionsgemisch löslich. Dadurch lassen sie sich im Chargenbetrieb durch einfache Filtration abtrennen und können wiederverwendet werden. Deshalb ist die verwendete Katalysatormenge nicht kritisch ; als günstig haben sich 10-20 Vol.-% bezogen auf eingesetztes Aceton erwiesen. Der Katalysator kann aber auch in einem Reaktor als Festbett angeordnet werden, wodurch eine kontinuierliche Arbeitsweise möglich wird.

Die Umsetzung wird wegen des niedrigen Siedepunktes des Acetons zweckmässigerweise unter Druck, unter Eigendruck des Systems, durchgeführt. Der Eigendruck kann auch durch Zugabe von Inertgasen erhöht werden. Geeignete Inertgase sind beispielsweise Stickstoff, Wasserstoff und Edelgase, wie Helium.

Für den Chargenbetrieb ergeben sich dabei Reaktionszeiten zwischen 0,2-6 Stunden, während bei kontinuierlicher Fahrweise LHSV-Werte (Raum/Zeit-Belastungswerte) von 0,2-10 l Aceton/l Kontakt · h vorliegen.

Neben Aceton können auch seine niedermolekularen Kondensationsprodukte, insbesondere Diacetonalkohol, Mesityloxid, und Umsetzungsprodukte mit Ammoniak, wie Diacetonamin, Acetonin und Triacetondiamin, eingesetzt werden. Soweit diese Produkte als Nebenprodukte anfallen, ist ihre Rückführung möglich.

Das neue Verfahren besitzt den Vorteil, dass Aceton und Ammoniak einstufig direkt und auch kontinuierlich unter umweltfreundlichen Bedingungen umgesetzt werden können. Ein weiterer Vorteil ist die Rückführungsmöglichkeit der entstehenden Zwischenprodukte in den Prozess, wodurch höhere Ausbeuten am Endprodukt, bezogen auf das umgesetzte Aceton, möglich werden.

Das 2,2,6,6-Tetramethylpiperidon-(4) ist ein wertvolles Ausgangsprodukt zur Herstellung von Stoffen mit Statilisatoreigenschaften.

Das Verfahren der vorliegenden Erfindung wird durch die nachstehenden Beispiele illustriert :

Beispiel 1

In einen 2 l Autoklav aus rostfreiem Stahl wurden 1,1 l Aceton und 200 ml eines sauren Ionenaustauschers auf der Basis eines Styrol-Divinylbenzolmischpolymerisats mit Sulfonsäureresten, der im Handel mit der Bezeichnung Lewatit® SP 120 erhältlich ist, eingefüllt. Es wurde mit Stickstoff gespult ; danach wurden 200 ml Ammoniak flüssig zugegeben. Unter intensivem Rühren wurde auf 100 °C aufgeheizt und 4 Stunden bei dieser Temperatur gehalten. Nach Abkühlen auf Raumtemperatur wurde entspannt und vom Ionenaustauscher abfiltriert. Es wurden 960 g Reaktionsprodukt erhalten. Die gaschromatographische Analyse ergab folgende Zusammensetzung :

45,5 %  Aceton
0,4 %  Mesityloxid
34,7 %  Diacetonamin
8,5 %  N-haltige Zwischenfraktionen
9,5 %  Triacetonamin
1,1 %  höhersiedende Anteile

Berücksichtigt man, dass Aceton, Mesityloxid und Diacetonamin in die Reaktions wieder eingesetzt werden können, beträgt der Umsatz 19,1 %, die Triacetonamin-Ausbeute dagegen 49,7 %, bezogen auf den Umsatz.

Beispiel 2

Entsprechend Beispiel 1 wurden 1,1 l Aceton und 200 ml Ammoniak umgesetzt. Als Katalysator wurden jedoch 200 ml Aluminiumhydrosilikat vom Montmorillonit-Typ, das im Handel unter der Bezeichnung K 10 erhältlich ist, eingesetzt und der Systemdruck nach Erreichen der Reaktionstemperatur mit Stickstoff auf 125 bar angehoben. Die gaschromatographische Analyse des Reaktionsgemisches ergab folgende Zusammensetzung :

33,8 %  Aceton
0,1 %  Mesityloxid
52,4 %  Diacetonamin
7,9 %  N-haltige Zwischenfraktionen
5,5 %  Triacetonamin
0,6 %  höhersiedende Anteile

Dieses entsprach einem Umsatz von 14,0 % und einer Triacetonaminausbeute von 39,3 %.

Beispiel 3

Entsprechend Beispiel 1 wurden 1,1 l Aceton und 200 ml Ammoniak umgesetzt. Als Katalysator wurden jedoch 200 ml Aluminiumsilikat vom Mordenit-Typ, im Handel unter der Bezeichnung Zeolon 100 erhältlich, eingesetzt.

Nach vierstündiger Reaktion bei 100 °C unter Eigendruck zeigte das Reaktionsgemisch nach gaschromatographischer Analyse folgende Zusammensetzung :

60,5 %  Aceton
0,1 %  Mesityloxid
18,2 %  Diacetonamin
12,8 %  N-haltige Zwischenfraktionen
7,4 %  Triacetonamin
0,9 %  höhersiedende Anteile

Bei einem Umsatz von 21,1 % entsprach dieses einer Triacetonaminausbeute von 35,1 %.

Beispiel 4

Entsprechend Beispiel 2 beschrieben wurden 1,1 l Aceton mit 75 ml flüssigem Ammoniak in

Gegenwart von 200 ml Aluminiumhydrosilikat vom Montmorillonit-Typ unter Eigendruck bei 80 °C umgesetzt. Durch gaschromatographische Analyse wurde folgende Zusammensetzung des Reaktionsgemisches ermittelt:

| | |
|---|---|
| 60,2 % | Aceton |
| 0,1 % | Mesityloxid |
| 17,3 % | Diacetonamin |
| 5,9 % | N-haltige Zwischenfraktionen |
| 15,6 % | Triacetonamin |
| 0,9 % | höhersiedende Anteile |

Bei einem Umsatz von 22,4 % betrug die Triacetonaminausbeute 69,6 %.

Beispiel 5

Ein zylindrischer Reaktor wurde mit Lewatit® SP 120 so gefüllt, dass dieses Katalysatormaterial durch Siebe abgetrennt zwischen zwei Lagen von Tonraschigringen angeordnet war. Das Schüttvolumen des Katalysators betrug 650 ml im feuchten Zustand, schrumpfte aber nach Behandlung mit Aceton auf 500 ml. Der Reaktor wurde mittels elektrischer Beheizung auf 100 °C Reaktionstemperatur gebracht und dann kontinuierlich mit 1 000 ml/h Aceton und 50 ml/h Ammoniak beschickt, indem die beiden Reaktanten aus Vorlagen flüssig in den unteren Reaktorteil gepumpt wurden. Das Reaktionsprodukt gelangte vom oberen Reaktorteil flüssig in einen Abschneider und wurde aus diesem kontinuierlichen abgezogen; das System wurde mit Inertgas ($H_2$) unter einem Druck von 70 bar gehalten.

Das Reaktionsprodukt besass laut GC-Analyse folgende Zusammensetzung:

| | |
|---|---|
| 64,5 % | Aceton |
| 4,6 % | Mesityloxid |
| 4,0 % | Diacetonamin |
| 7,6 % | N-haltige Zwischenfraktionen |
| 18,8 % | Triacetonamin |
| 0,5 % | Höhersieder |

Daraus ergab sich ein Umsatz von 26,9 %, der wie im Beispiel 1 errechnet wurde, sowie eine 69,9 %ige Ausbeute bezogen auf den Umsatz.

Nach 86 Tagen wurde der Versuch abgebrochen, obwohl keinerlei Beeinträchtigung der Umsatz- und Ausbeuteverhältnisse eingetreten war.

Beispiel 6

Die Reaktionskomponenten wurden unter sonst gleichen Bedingungen wie im Beispiel 5 oben in den Reaktor gepumpt, d. h. es wurde nach dem Rieselverfahren gearbeitet. Die dabei erhaltenen Resultate unterscheiden sich nicht von denen im Beispiel 5 aufgeführten.

Beispiel 5a bzw. 6a (Aufarbeitung der Verfahrensprodukte)

Die Aufarbeitung der nach Beispiel 5 und 6 erhaltenen Reaktionsprodukte erfolgte durch chargenweise Destillation. Zunächst wurde unter Normaldruck gearbeitet.

Dabei fiel als erste Fraktion Aceton an. Als zweite Fraktion ging Mesityloxid als Azeotrop mit dem Reaktionswasser über, das mittels Phasenscheider abgetrennt wurde. Als dritte Fraktion wurde reines Mesityloxid erhalten. Das im Reaktionsgemisch enthaltene Diacetonamin spaltete unter den vorliegenden Bedingungen in Ammoniak und Mesityloxid zurück. Die organischen Anteile dieser Fraktionen wurden wieder in die Reaktion eingesetzt. Anschliessend wird die Destillation unter Vakuum fortgesetzt. Es wurde eine N-haltige Zwischenfraktion erhalten, der die Triacetonamin-Reinfraktion ($Kp_{10}$ 78-82 °C; $n_D$ 20 1,463 0) folgte; sie entsprach 16,3 % des in die Destillation eingesetzten Produktes. Die N-haltige Zwischenfraktion un der Destillationsrückstand wurden verworfen.

Bei den Prozentangaben der vorstehenden Beispiele handelt es sich um Gewichtsprozente.

**Patentansprüche**

1. Verfahren zur einstufigen Herstellung von 2,2,6,6-Tetramethylpiperidon-(4) durch Umsetzung von Aceton und Ammoniak im Molverhältnis 2-25 : 1 in Gegenwart von festen sauren Katalysatoren bei erhöhter Temperatur, beim Eigendruck des Systems, der gegebenenfalls durch Inertgaszugabe erhöht ist, dadurch gekennzeichnet, dass man Aceton und Ammoniak bei Temperaturen im Bereich von 80-130 °C in Gegenwart von festen sauren Katalysatoren, die sowohl in den Einsatzprodukten als auch im Reaktionsgemisch praktisch unlöslich sind, umsetzt.

2. Verfahren nach Anspruch 1, dadurch gekennzeichnet, dass man einen Teil des Acetons durch niedermolekulare Kondensationsprodukte des Acetons ersetzt.

3. Verfahren nach Anspruch 1, dadurch gekennzeichnet, dass man einen Teil des Acetons durch niedermolekulare Umsetzungsprodukte des Acetons mit Ammoniak ersetzt.

4. Verfahren nach Anspruch 1, dadurch gekennzeichnet, dass man als festen, sauren Katalysator Aluminiumhydrosilikate vom Bentonit- und/oder Montmorillonit-Typ verwendet.

5. Verfahren nach Anspruch 1, dadurch gekennzeichnet, dass man als festen, sauren Katalysator einen stark sauren Kationenaustauscher auf Polystyrolbasis verwendet.

6. Verfahren nach Anspruch 1, dadurch gekennzeichnet, dass man als festen, sauren Katalysator einen anorganischen Kationenaustauscher auf Aluminiumsilikatbasis vom Zeolith-, Mordenit- und/oder Erionit-Typ verwendet.

7. Verfahren nach einem der Ansprüche 5 und 6, dadurch gekennzeichnet, dass man den festen, sauren Ionenaustauscher in der Ammoniumform verwendet.

**0 004 104**

## Claims

1. Process for the one step preparation of 2,2,6,6-tetramethylpiperidone-(4) by reaction of acetone and ammonia in a molar ratio of 2-25 : 1 in the presence of solid acid catalysts at elevated temperatures, at the self-pressure of the system, which is increased by addition of inert gas, if necessary, characterised in that acetone and ammonia are reacted at temperatures in the range of 80-130 °C in the presence of solid acid catalysts, which are as well practically insoluble in the feed stocks as also in the reaction mixture.

2. Process according to Claim 1, characterised in that part of the acetone is replaced by lower molecular condensation products of the acetone.

3. Process according to Claim 1, characterised in that part of the acetone is replaced by lower molecular reaction products of the acetone.

4. Process according to Claim 1, characterised in that as solid acid catalyst aluminium hydrosilicates of the bentonite- and/or montmorillonite-type are used.

5. Process according to Claim 1, characterised in that as solid acid catalyst a strong acid cation exchanger on basis of polystyrene is used.

6. Process according to Claim 1, characterised in that as solid acid catalyst an anorganic cation exchanger on the basis of aluminium silicate of zeolite-, mordenite- and/or erionite-type is used.

7. Process according to one of Claims 5 and 6, characterised in that the solid acid ion exchanger is used in the form of ammonia.

## Revendications

1. Procédé pour la fabrication de 2,2,6,6-tétra-méthylpipéridone-(4) par réaction d'acétone et de gaz ammoniac dans le rapport moléculaire 2 à 25 : 1, en une seule étape, en présence de catalyseurs acides solides, à température élevée, à la pression propre du système, éventuellement augmentée par addition de gaz inerte, procédé caractérisé en ce que l'on fait réagir l'acétone et l'ammoniac à des températures de l'ordre de 80 à 130 °C, en présence de catalyseurs acides, solides, qui sont pratiquement insolubles aussi bien dans les produits de départ que dans le mélange de réaction.

2. Procédé suivant la revendication 1, caractérisé en ce que l'on remplace une partie de l'acétone par des produits de condensation de l'acétone à faible poids moléculaire.

3. Procédé suivant la revendication 1, caractérisé en ce que l'on remplace une partie de l'acétone par des produits de condensation de l'acétone avec le gaz ammoniac à faibles poids moléculaires.

4. Procédé suivant la revendication 1, caractérisé en ce que l'on utilise, comme catalyseur acide, solide, des hydrosilicates d'aluminium du type bentonite et/ou montmorillonite.

5. Procédé suivant la revendication 1, caractérisé en ce que l'on utilise comme catalyseur acide, solide, un échangeur de cations fortement acide à base de polystyrène.

6. Procédé suivant la revendication 1, caractérisé en ce que l'on utilise, comme catalyseur acide, solide, un échangeur de cations minéral à base de silicate d'aluminium du type zéolithe, mordénite et/ou érionite.

7. Procédé suivant l'une des revendications 5 et 6, caractérisé en ce que l'on utilise l'échangeur d'ions acide, solide, sous la forme d'ammonium.